# FASCICULE DE BREVET EUROPEEN

(11) **EP 1 303 277 B1**
(45) Date de publication et mention de la délivrance du brevet: **01.08.2007**
(21) Numéro de dépôt: 01936591.5
(22) Date de dépôt: 18.05.2001
(51) Int. Cl.: A61K 31/46, A61P 25/30, A61K 9/68

(54) **COMPOSITION PHARMACEUTIQUE A BASE DE COCAETHYLENE ET SON UTILISATION POUR LE TRAITEMENT DE LA DEPENDANCE AUX SUBSTANCES PSYCHOACTIVES**
PHARMAZEUTISCHE ZUSAMMENSTELLUNG AUF BASIS VON COCAETHYLEN UND DESSEN VERWENDUNG ZUR BEHANDLUNG DER ABHÄNGIGKEIT VON PSYCHOAKTIVEN SUBSTANZEN
PHARMACEUTICAL COMPOSITION BASED ON COCAETHYLENE AND USE THEREOF FOR TREATING PSYCHOACTIVE SUBSTANCE DEPENDENCE

(30) Priorité: 18.07.2000 FR 0009408
(43) Date de publication de la demande: 23.04.2003
(73) Titulaire: Debussy Holding S.A., 2535 Luxembourg (LU)
(72) Inventeur: LOWENSTEIN, William, F-75007 Paris (FR); SANCHEZ, Mario, F-75003 Paris (FR); LEPERE-PREVOT, Bénédicte, F-75003 Paris (FR); DE ROTHSCHILD, Benjamin, CH-1292 Chambesy (CH)
(74) Mandataire: Benech, Frédéric
(86) Numéro de dépôt international: PCT/FR2001/001544
(87) Numéro de publication internationale: WO 2002/005817

(56) Documents cités:
- US-A- 4 512 996
- BRADBERRY CHARLES W ET AL: "Rapid induction of behavioral and neurochemical tolerance to cocaethylene, a model compound for agonist therapy of cocaine dependence." PSYCHOPHARMACOLOGY, vol. 146, no. 1, 1999, pages 87-92, XP000992247 ISSN: 0033-3158
- CARROLL, F. IVY ET AL: "Cocaine and 3.beta.-(4'-Substituted phenyl)tropane-2.beta.- carboxylic Acid Ester and Amide Analogs. New High-Affinity and Selective Compounds for the Dopamine Transporter" J. MED. CHEM. (1995), 38(2), 379-88, XP000990413
- SCHUELKE, GUY S. ET AL: "Cocaine analgesia: an in vivo structure-activity study" PHARMACOL., BIOCHEM. BEHAV. (1996), 53(1), 133-40, XP000992135
- MCCANCE E.F. ET AL: "Cocaethylene: Pharmacology, physiology and behavioral effects in humans." JOURNAL OF PHARMACOLOGY AND EXPERIMENTAL THERAPEUTICS, (1995) 274/1 (215-223)., XP000992238
- ELSWORTH JOHN D ET AL: "Serotonin involvement in cocaine sensitization: Clues from studies with cocaine analogs." DRUG DEVELOPMENT RESEARCH, vol. 30, no. 3, 1993, pages 189-200, XP000992240 ISSN: 0272-4391
- RAVEN M.A. ET AL: "Comparison of the reinforcing and anxiogenic effects of intravenous cocaine and cocaethylene." EXPERIMENTAL AND CLINICAL PSYCHOPHARMACOLOGY, (2000) 8/1 (117-124)., XP000992251
- HOROWITZ, JUDITH M. ET AL: "Cocaethylene: effects on brain systems and behavior" ADDICT. BIOL. (1999), 4(2), 127-140, XP000992235

## Description

La présente invention concerne une composition pharmaceutique contenant du cocaéthylène en tant qu'ingrédient actif ainsi que son utilisation pour traiter la dépendance aux substances psychoactives.

Il existe de nombreuses substances psychoactives telles que l'alcool, les amphétamines, la cocaïne, l'ecstasy, les dérivés opiacés comme l'opium ou l'héroïne, le LSD, le tabac ou le cannabis qui outre leurs effets néfastes sur l'organisme entraînent de fortes dépendances chez les usagers de ces substances.

Ces substances induisent des modifications à plusieurs niveaux du système nerveux central et notamment au niveau de l'ensemble des systèmes dopaminergique, sérotoninergique et noradrénergique. Il est reconnu que les déséquilibres -augmentation comme réduction- de ces systèmes sont par ailleurs à la base d'évolutions pathologiques comme la schizophrénie, les processus dépressifs ou maniaco-dépressifs et probablement impliqués dans la production de phénoménologies hallucinatoires. L'abus des substances psychoactives citées ci-dessus intervient et produit des moments dits féconds ou productifs dans les pathologies sus-citées.

Hormis la prise en charge de patients dépendants aux opiacés par des traitements dit « de maintenance » avec la méthadone et la buprénorphine, il n'existe pas pour l'instant de traitement agoniste spécifique dans le champ des addictions. De nombreuses pistes thérapeutiques ont été suivies telles que l'action agoniste-antagoniste des récepteurs dopaminergiques ou l'action sur le système sérotoninergique. De plus, pour la prise en charge de patients dépendants à la cocaïne, l'utilisation d'anticorps anti-cocaïne ou de vaccin anti-cocaïne a été envisagée.

Toutefois, il n'existe aucun traitement agoniste spécifique pour traiter la dépendance à la cocaïne.

Il apparaît alors important de mettre en évidence un composé actif permettant d'agir comme agoniste par rapport à l'action de la cocaïne, c'est-à-dire agissant sur les mêmes structures et dans le même sens, et de préparer des compositions pharmaceutiques contenant ce composé à titre d'agent actif pour traiter la dépendance aux substances psychoactives.

Le cocaéthylène se lie au transporteur de la dopamine, bloque ainsi la recapture de la dopamine et provoque donc une élévation du taux de la dopamine dans les structures du système nerveux central et notamment le système dopaminergique dont le noyau Accumbens.

La demande de brevet internationale WO 99/56747 décrit des compositions pharmaceutiques utilisées pour le traitement de la dépendance à l'héroïne, aux narcotiques, à la cocaïne, aux amphétamines et à la marihuana, contenant à titre d'ingrédients actifs deux composants neuroleptiques utilisés simultanément : AMPT (alpha-méthyl-para-tyrosine) et Haldoperidol, qui est un composé de la série des butyrophénones. L'Haloperidol agit en tant qu'antagoniste des récepteurs dopaminergiques post-synaptiques et l'AMPT agit en tant que régulateur négatif du récepteur à la dopamine.

La demande de brevet WO 97/42950 décrit une méthode de traitement de la dépendance à la cocaïne et aux amphétamines par l'utilisation d'un composé dérivé du benzomorphane.

On connaît également les résultats de recherches effectuées sur les effets similaires entre la cocaïne et le cocaéthylène (BRADBERRY CHARLES W ET AL : « Rapid induction of behavioral and neurochemical tolerance to cocaethylene, a model compound for agonist therapy of cocaine dépendance. « PSYCHOPHARMACOLOGT, vol. 146, no.1, 1999, pages 87-92, XP000992247 ISSN : 0033-3158).

On connaît aussi le traitement de l'arthrite avec du benzoylecgonine (US-A-4 512 996) ou encore l'importance de la connaissance de la sélectivité du ligaud en cas de traitement de substitution de la cocaïne ( CARROLL, F. IVY ET AL : « Cocaine and 3.beta,-(4'-Substituted phenyl)tropane-2.beta.-carboxylic Acid Ester and Amide Analogs. New High-Affinity and Selective Compounds for the Dopamine Transporter » J. MED. CHEM. (1995), 38(2), 379-88, XP000990413).

Enfin le document SCHUELKE, GUY S. ET AL : « Cocaine analgesia : an in vivo structure-activity study » PHARMACOL., BIOCHEM. BEHAV. (1996), 53(1), 133-40, XP000992135 s'intéresse à la formulation possible d'un traitement ponctuel à l'intoxication en cas d'abus de cocaïne.

Aucun de ces arts antérieurs ne décrit de traitement en continu par injection de cocaéthylène par le patient.

Les demandeurs ont découvert de manière surprenante que le cocaéthylène ou ester éthylique de la benzoylecgonine pouvait être utilisé en tant que substance active dans une composition pharmaceutique, en particulier pour traiter la dépendance aux substances psychoactives.

La présente invention concerne l'administration à l'homme, sous une forme galénique acceptable de gomme à mâcher, de cocaéthylène ou ester éthylique de la benzoylecgonine en tant qu'agent thérapeutique. La présente invention a ainsi pour objet une composition pharmaceutique et un médicament contenant à titre d'agent actif l'ester éthylique de la benzoylecgonine ainsi que leurs utilisations pour traiter la dépendance aux substances psychoactives.

La présente invention a pour principal objet une composition pharmaceutique comprenant à titre d'ingrédient actif, l'ester éthylique de la benzoylecgonine sous forme de base libre ou de ses sels pharmaceutiquement acceptables, dans un milieu pharmaceutiquement acceptable caractérisé en ce que elle est sous forme de gomme à mâcher.

La composition pharmaceutique peut contenir de 0,1 à 50 %, et préférentiellement de 0,5 à 10 % en poids d'ester éthylique de la benzoylecgonine par rapport au poids total de la composition.

L'ester éthylique de la benzoylecgonine peut être sous forme de base libre ou de ses sels pharmaceutiquement acceptables généralement utilisés en pharmacie.

Les sels pharmaceutiquement acceptables sont choisis par exemple, parmi les sels d'acides minéraux ou organiques tels que les chlorhydrate, bromhydrate, nitrate, sulfate, phosphate, formiate, acétate, trifluoroacétate, fumarate, oxalate, tartrate, maléate, citrate, succinate, malate, méthanesulfonate, benzènesulfonate et p-toluènesulfonate, parmi les sels d'acides aminés tels que l'arginine, la lysine, l'omithine, l'acide aspartique et l'acide glutamique, ou parmi les catiorésines carboxylate ou sulfonate.

La catiorésine est une résine échangeuse d'ions capable de lier des ions chargés positivement (cations). On peut utiliser, outre la catiorésine, tout autre composé permettant un relarguage lent du cocaéthylène dans l'organisme du patient.

L'ester éthylique de la benzoylecgonine peut donc se trouver par exemple sous forme de catiorésine carboxylate.

La composition pharmaceutique peut en outre contenir des excipients tels que le lactose, le sucrose, le D-mannitol, la cellulose cristalline, l'amidon ou tout autre type d'excipients généralement utilisés en pharmacie et des adjuvants pharmaceutiquement acceptables tels que des agents mouillants, isotonisants, émulsifiants, dispersants, stabilisants, solvants, liants, aromatisants, plastifiants, anti-oxydants, substances de mastication, colorants, conservateurs, tampons, adoucissants, ou tout autre type d'adjuvants généralement utilisés en pharmacie.

Les quantités de ces différents adjuvants sont celles classiquement utilisées dans le domaine considéré.

Bien entendu, l'homme de l'art veillera à choisir le ou les éventuels composés à ajouter à la composition selon l'invention, de manière telle que les propriétés avantageuses attachées intrinsèquement à la composition conforme à l'invention ne soient pas, ou substantiellement pas, altérées par l'addition envisagée.

La présente invention a également pour objet un médicament pour traiter la dépendance aux substances psychoactives, comprenant à titre d'ingrédient actif l'ester éthylique de la benzoylecgonine sous forme de base libre ou de ses sels pharmaceutiquement acceptables, dans un milieu pharmaceutiquement acceptable.

Le médicament peut être utilisé pour traiter la dépendance aux substances psychoactives et notamment la cocaïne, l'alcool, les amphétamines, l'ecstasy, l'héroïne, la morphine, le LSD, le cannabis, le tabac et les narcotiques.

La dose quotidienne de médicament a administrer est variable selon le niveau de la dépendance, la méthode d'administration et le poids du patient. Le dosage unitaire de cette forme administrée à l'homme est compris entre 2 et 1000 milligrammes et de préférence entre 5 et 500 milligrammes par unité de prise. Il peut y avoir entre 1 et 20 prises par jour.

Selon un mode préféré de l'invention, le médicament peut être utilisé pour traiter la dépendance à la cocaïne.

Le médicament peut également être utilisé pour traiter les affections concomitantes à l'usage de substances psychoactives, dans le cadre de moments féconds ou productifs des pathologies comme la schizophrénie, la psychose maniaco-dépressive, la paranoïa ou les phénomènes hallucinatoires. Il peut du reste être combiné aux inhibiteurs de récepteurs de la dopamine et/ou aux inhibiteurs de la recapture de la sérotonine, en association avec des dérivés opiacés utilisés en thérapeutique ou tout autre traitement régulateur thymique.

La présente invention a également pour objet l'utilisation du cocaéthylène pour la préparation d'un médicament sous forme de gomme à mâcher pour traiter la dépendance aux substances psychoactives et plus particulièrement pour traiter la dépendance à la cocaïne, à l'alcool, aux amphétamines, à l'ecstasy, à l'héroïne, à la morphine, au LSD, au cannabis, au tabac ou aux narcotiques et encore plus préférentiellement pour traiter la dépendance à la cocaïne.

Le cocaéthylène peut aussi être utilisé sous forme de gomme à mâcher pour la préparation d'un médicament pour traiter les affections mentales concomitantes à l'usage de substances psychoactives dans le cadre de la schizophrénie, de la psychose maniaco-dépressive, de la paranoïa et autres psychoses à forme hallucinatoire.

Les exemples qui suivent sont destinés à illustrer l'invention.

### Exemples de formulations contenant du cocaéthylène

### Exemple 1

### Gomme à mâcher médicamenteuse

| | |
|---|---|
| Cocaéthylène (P.A.) sous forme de catiorésine carboxylate | 10 mg P.A. |
| Bicarbonate de sodium | 10 mg |
| Carbonate de sodium anhydre | 20 mg |
| Gomme Dreyco^{®} | 750 mg |
| Sorbitol | 160 mg |
| Sorbitol à 70 % cristallisable | 40 mg |
| Glycérol à 85 % | 10 mg |
| Aromatisants en quantité suffisante | |

La gomme Dreyco^{®} est composée de :
- substances synthétiques de mastication : copolymère d'isobutylène et d'isopropène, cire dérivée du pétrole, acétate de polyvinyle, polyéthylène, polyisobutylène.
- plastifiants : esters glycériques de colophane, esters glycériques de colophane polymérisée, huiles végétales hydrogénées, monostéarate de glycérol.
- substances insolubles dans l'eau : carbonate de calcium.
- antioxydants : butyl-hydroxy-toluène.

## Revendications

1. Composition pharmaceutique comprenant à titre d'ingrédient actif, l'ester éthylique de la benzoylecgonine sous forme de base libre ou de ses sels pharmaceutiquement acceptables, dans un milieu pharmaceutiquement acceptable, **caractérisée en ce qu'**elle est sous la forme de gomme à mâcher.

2. Composition selon la revendication 1, **caractérisée en ce qu'**elle contient de 0, 1 à 50% en poids d'ester éthylique de la benzoylecgonine par rapport au poids total de la composition.

3. Composition selon la revendication 1 ou 2, **caractérisée en ce qu'**elle contient de 0,5 à 10% en poids d'ester éthylique de la benzoylecgonine par rapport au poids total de la composition.

4. Composition selon l'une quelconque des revendications 1 à 3, **caractérisée en ce que** le sel pharmaceutiquement acceptable est choisi parmi les sels d'acides minéraux ou organiques tels que les chlorhydrate, bromhydrate, nitrate, sulfate, phosphate, formiate, acétate, trifluoroacétate, fumarate, oxalate, tartrate, maléate, citrate, succinate, malate, méthanesulfonate, benzènesulfonate, p-toluènesulfonate, parmi les sels d'acides aminés et parmi les catiorésines carboxylate ou sulfonate.

5. Composition selon la revendication 4, **caractérisée en ce que** l'ester éhylique de la benzoylecgonine est sous forme de catiorésine carboxylate.

6. Composition selon l'une quelconque des revendications 1 à 5, **caractérisée en ce qu'**elle contient des excipients et des adjuvants pharmaceutiquement acceptables.

7. Médicament pour traiter la dépendance aux substances psychoactives, comprenant à titre d'ingrédient actif l'ester éthylique de la benzoylecgonine sous forme de base libre ou de ses sels pharmaceutiquement acceptables, dans un milieu pharmaceutiquement acceptable, administré sous forme de gomme à mâcher.

8. Médicament selon la revendication 7 pour traiter la dépendance à la cocaïne, à l'alcool, aux amphétamines, à l'ecstasy, à l'héroïne, à la morphine, au LSD, au canabis, au tabac ou aux narcotiques.

9. Médicament selon la revendication 7 ou 8 pour traiter la dépendance à la cocaïne.

10. Médicament pour traiter les affections mentales concomitantes à l'usage de substances psychoactives dans le cadre des pathologies telles que la schizophrénie, la psychose maniaco-dépressive et les phénomènes hallucinatoires, comprenant à titre d'ingrédient actif l'ester éthylique de la benzoylecgonine ou l'un de ses sels pharmaceutiquement acceptables, administré sous forme de gomme à mâcher.

11. Utilisation de l'ester éthylique de la benzoylecgonine ou l'un de ses sels pharmaceutiquement acceptables pour la préparation d'un médicament pour traiter la dépendance aux substances psychoactives administré sous forme de gomme à mâcher.

12. Utilisation selon la revendication 11, pour traiter la dépendance à la cocaïne, à l'alcool, aux amphétamines, à l'ecstasy, à l'héroïne, à la morphine, au LSD, au cannabis, au tabac ou aux narcotiques.

13. Utilisation selon la revendication 11 ou 12, pour traiter la dépendance à la cocaïne.

14. Utilisation de l'ester éthylique de la benzoylecgonine ou l'un de ses sels pharmaceutiquement acceptables pour la préparation d'un médicament administré sous forme de gomme à mâcher, pour traiter les affections mentales concomitantes à l'usage de substances psychoactives dans le cadre de la schizophrénie, de la psychose maniaco-dépressive, de la paranoïa et autres psychoses à forme hallucinatoire.

## Claims

1. Pharmaceutical composition comprising, as active ingredient, the ethyl ester of benzoylecgonine in the form of a free base or its pharmaceutically acceptable salts, in a pharmaceutically acceptable medium, **characterised in that** it is in the form of chewing gum.

2. Composition according to Claim 1, **characterised in that** it contains from 0.1 to 50% by weight of ethyl ester of benzoylecgonine in relation to the total weight of the composition.

3. Composition according to Claim 1 or 2, **characterised in that** it contains from 0.5 to 10% by weight of ethyl ester of benzoylecgonine in relation to the total weight of the composition.

4. Composition according to any one of Claims 1 to 3, **characterised in that** the pharmaceutically acceptable salt is selected from among organic or mineral acid salts such as hydrochloride, hydrobromide, nitrate, sulphate, phosphate, formate, acetate, trifluoroacetate, fumarate, oxalate, tartrate, maléate, citrate, succinate, malate, methanesulphonate, benzenesulphonate, p-toluenesufphonate, from among amino acid salts and the cation exchange resins carboxylate or sulphonate.

5. Composition according to Claim 4, **characterised in that** the ethyl ester of benzoylecgonine is in the form of carboxylate cation exchange resin.

6. Composition according to any one of Claims 1 to 5, **characterised in that** it contains pharmaceutically acceptable additives and excipients.

7. Medicinal product for treating dependence on psychoactive substances, comprising, as active ingredient, the ethyl ester of benzoylecgonine in the form of free base or its pharmaceutically acceptable salts, in a pharmaceutically acceptable medium, administered in the form of chewing gum.

8. Medicinal product according to Claim 7 for treating dependence on cocaine, alcohol, amphetamines, ecstasy, heroin, morphine, LSD, cannabis, tobacco or narcotics.

9. Medicinal product according to Claim 7 or 8 for treating dependence on cocaine.

10. Medicinal product for treating mental disorders concomitant with the use of psychoactive substances in the context of pathologies such as schizophrenia, manic depression and hallucinatory phenomena, comprising, as active ingredient, the ethyl ester of benzoylecgonine or one of its pharmaceutically acceptable salts, administered in the form of chewing gum.

11. Use of the ethyl ester of benzoylecgonine or one of its pharmaceutically acceptable salts for the preparation of a medicinal product for treating dependence on psychoactive substances administered in the form of chewing gum.

12. Use according to Claim 11, for treating dependence on cocaine, alcohol, amphetamines, ecstasy, heroin, morphine, LSD, cannabis, tobacco or narcotics.

13. Use according to Claim 11 or 12, for treating dependence on cocaine.

14. Use of the ethyl ester of benzoylecgonine or one of its pharmaceutically acceptable salts for the preparation of a medicinal product administered in the form of chewing gum for treating mental disorders concomitant with the use of psychoactive substances in the context of schizophrenia, manic depression, paranoia and other hallucinatory-type psychoses.

## Patentansprüche

1. Pharmazeutische Zusammenstellung enthaltend als aktiven Wirkstoff den Ethylester von Benzoylecgonin in Form der freien Base oder eines pharmazeutisch akzeptablen Salzes davon in einem pharmazeutisch akzeptablen Milieu,
**dadurch gekennzeichnet,**
**dass** sie in Form eines Kaugummis vorliegt.

2. Zusammenstellung nach Anspruch 1,
**dadurch gekennzeichnet,**
**dass** sie 0,1 bis 50 Gewichtsprozent Ethylester von Benzoylecgonin bezogen auf das Gesamtgewicht der Zusammenstellung enthält.

3. Zusammenstellung nach Anspruch 1 oder 2,
**dadurch gekennzeichnet,**
**dass** sie 0,5 bis 10 Gewichtsprozent Ethylester von Benzoylecgonin bezogen auf das Gesamtgewicht der Zusammenstellung enthält.

4. Zusammenstellung nach einem der Ansprüche 1 bis 3,
**dadurch gekennzeichnet,**
**dass** das pharmazeutisch akzeptable Salz ausgewählt ist unter einem Salz einer Mineralsäure oder organischen Säure, wie einem Chlorhydrat, Bromhydrat, Nitrat, Sulfat, Phosphat, Formiat, Acetat, Trifluracetat, Fumarat, Oxalat, Tartrat, Maleat, Citrat, Succinat, Malat, Methansulfonat, Benzolsulfonat, p-Toluolsulfonat, unter Salzen von Aminosäuren, unter kationischen Carboxylatharzen oder kationischen Sulfonatharzen.

5. Zusammenstellung nach Anspruch 4,
**dadurch gekennzeichnet,**
**dass** der Ethylester von Benzoylecgonin in Form eines kationischen Carboxylatharzes vorliegt.

6. Zusammenstellung nach einem der Ansprüche 1 bis 5,
**dadurch gekennzeichnet,**
**dass** sie pharmazeutisch akzeptable Trägerstoffe und Hilfsstoffe enthält.

7. Arzneimittel zur Behandlung der Abhängigkeit von psychoaktiven Substanzen enthaltend als aktiven Wirkstoff den Ethylester von Benzoylecgonin in Form der freien Base oder von pharmazeutisch akzeptablen Salzen in einem pharmazeutisch akzeptablen Milieu zur Verabreichung in Form von Kaugummi.

8. Arzneimittel nach Anspruch 7,
zur Behandlung der Abhängigkeit von Kokain, Alkohol, Amphetaminen, Extasy, Heroin, Morphium, LSD, Cannabis, Tabak oder von Narkotika.

9. Arzneimittel nach Anspruch 7 oder 8,
zur Behandlung der Abhängigkeit von Kokain.

10. Arzneimittel zur Behandlung von Geisteszuständen, die mit dem Gebrauch von psychoaktiven Substanzen einhergehen, im Rahmen von Pathologien wie Schizophrenie, manisch-depressiver Erkrankung, halluzinatorischen Phänomenen, enthaltend als aktiven Wirkstoff den Ethylester von Benzoylecgonin oder ein pharmazeutisch akzeptables Salz zur Verabreichung in Form von Kaugummi.

11. Verwendung des Ethylesters von Benzoylecgonin oder eines pharmazeutisch akzeptablen Salzes davon zur Herstellung eines Medikaments zur Behandlung der Abhängigkeit von psychoaktiven Substanzen zur Verabreichung in Form von Kaugummi.

12. Verwendung nach Anspruch 11,
zur Behandlung der Abhängigkeit von Kokain, Alkohol, Amphetaminen, Extasy, Heroin, Morphium, LSD, Cannabis, Tabak oder Narkotika.

13. Verwendung nach Anspruch 11 oder 12,
zur Behandlung der Abhängigkeit von Kokain.

14. Verwendung des Ethylesters von Benzoylecgonin oder eines pharmazeutisch akzeptablen Salzes davon zur Herstellung eines Medikaments zur Verabreichung in Form von Kaugummi zur Behandlung von Geisteskrankheiten, die einhergehen mit dem Gebrauch von psychoaktiven Substanzen im Rahmen von Schizophrenie, manisch depressiver Psychose, Paranoia und anderen halluzinatorischen Psychosen.
